# EUROPÄISCHE PATENTANMELDUNG

(11) **EP 4 074 722 A1**
(43) Veröffentlichungstag der Anmeldung: **19.10.2022**
(21) Anmeldenummer: 21168825.4
(22) Anmeldetag: 16.04.2021
(51) Int. Cl.: C07F 9/6574, B01J 31/00, C07B 41/06, C07C 45/00

(54) **DIPHOSPHITE MIT EINEM OFFENEN, 3-METHYLIERTEN FLÜGELBAUSTEIN**

(71) Anmelder: Evonik Operations GmbH, 45128 Essen (DE)
(72) Erfinder: SALE, Anna Chiara, 45657 Recklinghausen (DE); FRANKE, Robert, 45772 Marl (DE); BRÄCHER, Alexander, 45721 Haltern am See (DE); FRIDAG, Dirk, 45721 Haltern am See (DE); MARKOVIC, Ana, 45721 Haltern am See (DE); KUCMIERCZYK, Peter, 44628 Herne (DE); KNOSSALLA, Johannes, 46514 Gahlen (DE)
(74) Vertreter: Evonik Patent Association

(57) **Zusammenfassung**

Diphosphite mit einem offenen, 3-methylierten Flügelbaustein und deren Verwendung in der Hydroformylierung.

## Beschreibung

Die Erfindung betrifft Diphosphite mit einem offenen, 3-methylierten Flügelbaustein und deren Verwendung in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

In EP 0213639 A2 ist auf Seite 98 in Beispiel 10 die folgende Verbindung dargestellt:

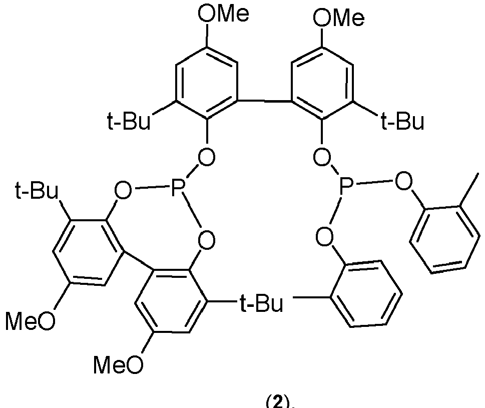

Die Verbindung (2) wird hier als Ligand in der Hydroformylierung von 1-Buten eingesetzt.

Die technische Aufgabe der Erfindung ist die Bereitstellung neuer Liganden, welche in der Hydroformylierung von Olefinen eine gesteigerte n/iso-Selektivität verglichen zu dem aus dem Stand der Technik bekennten Liganden aufweisen.

Die Aufgabe wird gelöst durch eine Verbindung gemäß Anspruch 1.

Verbindung gemäß der Struktur (I): 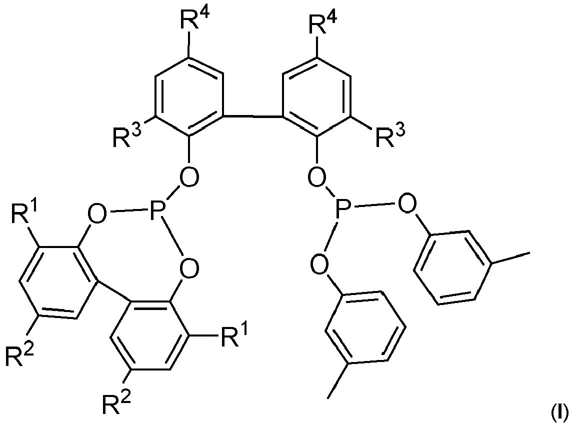 wobei R¹, R², R³, R⁴ ausgewählt sind aus: -H, -(C₁-C₁₂)-Alkyl, -O-(C₁-C₁₂)-Alkyl.

In einer Ausführungsform sind R¹, R³ ausgewählt aus: -H, -(C₁-C₁₂)-Alkyl.

In einer Ausführungsform stehen R¹, R³ für -(C₁-C₁₂)-Alkyl.

In einer Ausführungsform stehen R¹, R³ für -*t*Bu.

In einer Ausführungsform stehen R¹, R³ für den gleichen Rest.

In einer Ausführungsform sind R², R⁴ ausgewählt aus: -H, -O-(C₁-C₁₂)-Alkyl.

In einer Ausführungsform stehen R², R⁴ für -O-(C₁-C₁₂)-Alkyl.

In einer Ausführungsform stehen R², R⁴ für -OMe.

In einer Ausführungsform stehen R², R⁴ für den gleichen Rest.

In einer Ausführungsform weist die Verbindung die Struktur (1) auf:

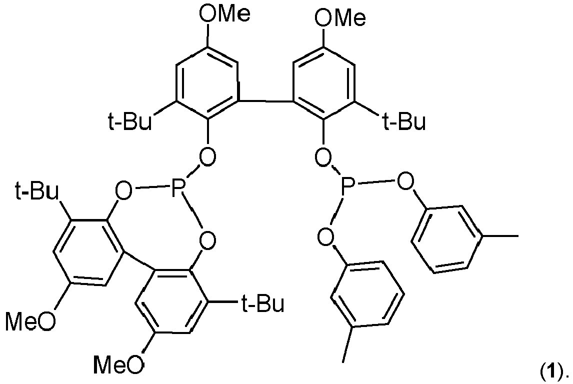

Neben der Verbindung als solche wird auch deren Verwendung zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Des Weiteren wird ein Verfahren beansprucht, in welchem die zuvor beschriebene Verbindung als Ligand eingesetzt wird.

Verfahren umfassend die Verfahrensschritte:
a) Vorlegen eines Olefins,
b) Zugabe einer zuvor beschriebenen Verbindung und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

In einer bevorzugten Ausführungsform ist das Metall Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 160 °C und ein Druck von 10 bis 60 bar. Besonders bevorzugt sind eine Temperatur von 100 °C bis 140 °C und ein Druck von 20 bis 50 bar. Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Di-n-buten, Di-iso-buten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Mit dem erfindungsgemäßen Verfahren können unter Verwendung der erfindungsgemäßen Liganden α-Olefine, endständig verzweigte, innenständige und innenständig verzweigte Olefine hydroformyliert werden.

Im Folgenden soll die Erfindung anhand von Ausführungsbeispielen näher erläutert werden.

### Arbeitsvorschriften

### Allgemeine Analytik

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet.

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR³¹P = SR¹H * (BF³¹P / BF¹H) = SR¹H * 0,4048.

### Synthese (1)

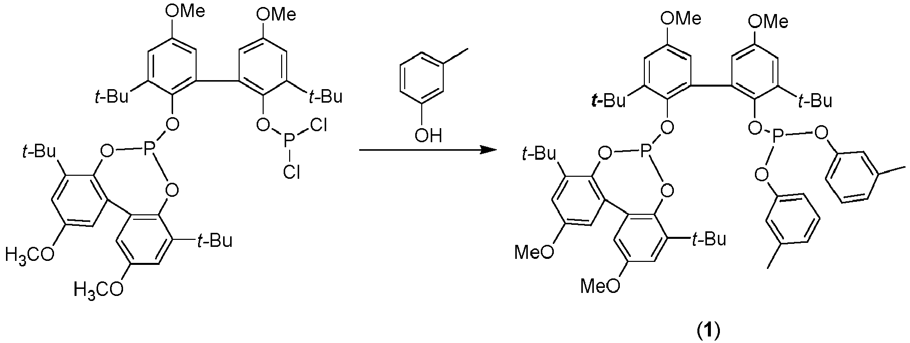

In der Glove-Box wurde in einem sekurierten 250 mL Schlenk 9 g (0,01 mol) Diorganophosphitdichlorphosphit eingewogen, anschließend ausgeschleusst und in 75 mL getrocknetem Toluol gelöst. In einem zweiten sekurierten 250 mL Schlenk wurden 2.2 g (2,1 mL 0,02 mol) 3-Methylphenol abgewogen und 12 h mittels Ölpumpenvakuum bei Raumtemperatur nachgetrocknet. Es wurde unter Rühren 50 mL getrocknetes Toluol und 3 mL = 2,2 g (0,022 mol) entgastes Triethylamin zugegeben und aufgelöst. Zur Phenol-Triethylamin Lösung wurde bei Raumtemperatur innerhalb 1,5 h das Dichlorophosphit hinzugegebern. Die Reaktion wurde 15 h bei Raumtemperatur gerührt und 1,5 mL (0,011 mol) Triethylamin zugegeben. Die Reaktionsmischung wurde dann noch 1,5 h bei 80 °C gerührt. Nach abkühlen auf Raumtemperatur, wurde der Ammoniumhydrochlorid Feststoff abgefrittet und das Filtrat mittels Ölpumpenvakuum bei 40 °C eingeengt. Der erhaltene Feststoff wurde 15 h mittels Ölpumpenvakuum bei Raumtemperatur nachgetrocknet. Ausbeute: 76%, Reinheit: 90%.

### Synthese (2) (Vergleichsligand)

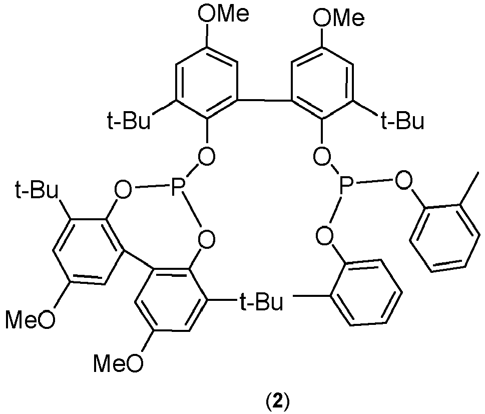

In der Glove-Box wurde in einem sekurierten 250 mL Schlenk 9 g (0,01 mol) Diorganophosphitdichlorphosphit eingewogen, anschließend ausgeschleusst und in 75 mL getrocknetem Toluol gelöst. In einem zweiten sekurierten 250 mL Schlenk wurden 2.2 g (2,1 mL 0,02 mol) 2-Methylphenol abgewogen und 12 h mittels Ölpumpenvakuum bei Raumtemperatur nachgetrocknet. Es wurde unter Rühren 50 mL getrocknetes Toluol und 3 mL = 2,2 g (0,022 mol) entgastes Triethylamin zugegeben und aufgelöst. Zur Phenol-Triethylamin Lösung wurde bei Raumtemperatur innerhalb 1,5 h das Dichlorophosphit hinzugegeben. Die Reaktionsmischung wurde 2 h bei Raumtemperatur gerührt und danach auf 80 °C erhitzt. Die Reaktionsmischung wurde 15 h bei dieser Temperatur gerührt und es wurden 3 mal 1,5 mL (0,011 mol) Triethylamin nachdosiert und 15 h weiter rühren gelassen. Das Ammoniumhydrochlorid wurde abgefrittet, 1 x mit 10 mL getrocknetem Toluol nachgewaschen bis zur Trockne eingeengt. Der Feststoff wurde 15 h bei Raumtemperatur getrocknet und mit 40 mL entgastem Acetonitril gerührt. Der ausgefallene weiße Feststoff wurde abgefrittet, der Schlenk wurde mit 2 mal 10 mL ACN nachgespült und nach dem Trocknen in die Glove-Box eingeschleust. Ausbeute 90%, Reinheit: 95%.

### Katalyseversuche

Die Hydroformylierung wurde in einem mit Druckkonstanthaltung, Gasflussmessung, und Begasungsrührer ausgestatteten 16 ml-Autoklaven der HEL group, Hertfordshire, Großbritannien, durchgeführt. Das als Substrat eingesetzte n-Octen (Oxeno GmbH, Octenisomerengemisch aus 1-Octen: 3 %; cis+trans-2-Octen: 49 %; cis+trans-3-Octen: 29 %; cis+trans-4-Octen: 16 %; gerüstisomere Octene: 3 %) wurde mehrere Stunden über Natrium am Rückfluss erhitzt und unter Argon destilliert.

Für die Versuche wurden die Reaktionslösungen vorab unter Argonatmosphäre vorbereitet. Hierfür wurden 0,0021 g Rh(acac)(CO)₂ und die entsprechende Menge an Phosphitverbindung eingewogen und mit 8,0 ml Toluol aufgefüllt. Die jeweils eingebrachte Masse an Toluol wurde für die GC-Analyse bestimmt. Anschließend wurden 1,80 g n-Octen (16 mmol) hinzugegeben. Die vorbereiteten Lösungen wurden anschließend in den Autoklaven eingefüllt und dieser dreimal mit Argon und dreimal mit Synthesegas (Linde; H₂ (99,999 %) : CO (99,997%) = 1:1) gespült. Dann wurde der Autoklav bei einem Gesamtdruck von 10 bar unter Rühren (900 U/min) auf die angestrebte Temperatur aufgeheizt. Nach Erreichen der Reaktionstemperatur wurde der Synthesegasdruck auf 20 bar erhöht und die Reaktion bei konstantem Druck für 4 h geführt. Der Autoklav wurde nach Ablauf der Reaktionszeit auf Raumtemperatur abgekühlt, unter Rühren entspannt und mit Argon gespült. Jeweils 0,5 ml der Reaktionsmischungen wurden nach Beenden der Reaktion entnommen, mit 4 ml Pentan verdünnt und gaschromatographisch analysiert: HP 5890 Series II plus, PONA, 50 m x 0,2 mm x 0,5 µm. Die quantitative Bestimmung von Restolefin und Aldehyd erfolgte gegen das Lösungsmittel Toluol als interner Standard.

### Ergebnisse der Katalyseversuche

### Reaktionsbedingungen:

[Rh]: 120 ppm, L:Rh = 1:2, p: 20 bar, T: 120 °C; t: 4 h

**Tabelle 1: Hydroformylierung der n-Octene**

| Ligand | n/iso-Selektivität in % |
|---|---|
| **1*** | 77 |
| **2** | 56 |

| | |
|---|---|
| * erfindungsgemäße Verbindung | |

### Definition der Selektivität:

Bei der Hydroformylierung gibt es die n/iso-Selektivität: das Verhältnis von linearem Aldehyd (= n) zu verzweigtem Aldehyd (= iso). Hierbei bedeutet die Selektivität bezüglich des n-Aldehyden, dass diese Menge an linearem Produkt gebildet wurde. Die restlichen Prozente entsprechen dann dem verzweigten Isomer. Bei einer Regioselektivität von 50% entstehen also n-Aldehyd und iso-Aldehyd zu gleichen Teilen.

Mit der erfindungsgemäßen Verbindung (1) konnte eine gegenüber dem Vergleichsliganden (2) gesteigerte n/iso-Selektivität erzielt werden.

Die durchgeführten Versuche belegen, dass die gestellte Aufgabe durch die erfindungsgemäße Verbindung gelöst wird.

## Patentansprüche

1. Verbindung gemäß der Struktur (**I**): 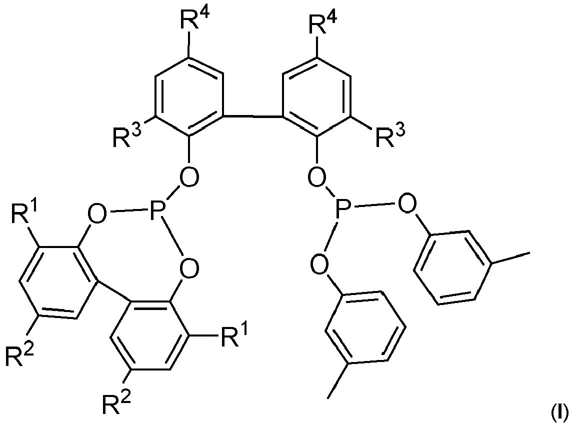 wobei R¹, R², R³, R⁴ ausgewählt sind aus: -H, -(C₁-C₁₂)-Alkyl, -O-(C₁-C₁₂)-Alkyl.

2. Verbindung nach Anspruch 1,
wobei R¹, R³ ausgewählt sind aus: -H, -(C₁-C₁₂)-Alkyl.

3. Verbindung nach einem der Ansprüche 1 oder 2,
wobei R¹, R³ für -(C₁-C₁₂)-Alkyl stehen.

4. Verbindung nach einem der Ansprüche 1 bis 3,
wobei R¹, R³ für den gleichen Rest stehen.

5. Verbindung nach einem der Ansprüche 1 bis 4,
wobei R², R⁴ ausgewählt sind aus: -H, -O-(C₁-C₁₂)-Alkyl.

6. Verbindung nach einem der Ansprüche 1 bis 5,
wobei R², R⁴ für -O-(C₁-C₁₂)-Alkyl stehen.

7. Verbindung nach einem der Ansprüche 1 bis 6,
wobei R², R⁴ für den gleichen Rest stehen.

8. Verbindung nach einem der Ansprüche 1 bis 7,
wobei die Verbindung die Struktur (1) aufweist:

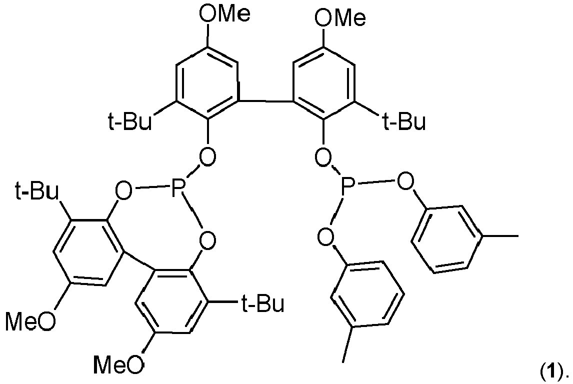

9. Verwendung einer Verbindung nach einem der Ansprüche 1 bis 8,
in einem Ligand-Metall-Komplex zur Katalyse einer Hydroformylierungsreaktion.

10. Verfahren umfassend die Verfahrensschritte:
a) Vorlegen eines Olefins,
b) Zugabe einer Verbindung nach einem der Ansprüche 1 bis 8 und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.
